# NEUE EUROPÄISCHE PATENTSCHRIFT

(11) **EP 0 359 773 B2**
(45) Veröffentlichungstag und Bekanntmachung des Hinweises auf die Entscheidung über den Einspruch: **18.12.2002**
(45) Hinweis auf die Patenterteilung: 06.10.1993
(21) Anmeldenummer: 88904956.5
(22) Anmeldetag: 21.05.1988
(51) Int. Cl.: A61B 19/00, A61B 6/02

(54) **VORRICHTUNG ZUR REPRODUZIERBAREN OPTISCHEN DARSTELLUNG EINES CHIRURGISCHEN EINGRIFFES**
DEVICE FOR OPTICAL REPRESENTATION OF SURGICAL OPERATIONS
DISPOSITIF DE REPRESENTATION OPTIQUE REPRODUISIBLE D'INTERVENTIONS CHIRURGICALES

(30) Priorität: 27.05.1987 DE 3717871
(43) Veröffentlichungstag der Anmeldung: 28.03.1990
(73) Patentinhaber: Surgical Navigation Technologies, Inc., Broomfield, CO 80020 (US)
(72) Erfinder: MÖSGES, Ralph, D-8000 München 40 (DE); MEYER-EBRECHT, Dietrich, D-5100 Aachen (DE); MOLL, Philipp, D-5100 Aachen (DE); SCHLÖNDORFF, Georg Dr., D-5106 Roetgen (DE)
(74) Vertreter: Klein, Friedrich
(86) Internationale Anmeldenummer: EP8800457
(87) Internationale Veröffentlichungsnummer: WO88009151

(56) Entgegenhaltungen:
- EP-A- 0 018 166
- WO-A-88/09151
- DE-A- 2 534 516
- DE-A- 2 852 949
- DE-A- 3 205 085
- DE-B- 1 303 213
- JP-A- 62 327
- US-A- 4 068 556
- US-A- 4 358 856
- US-A- 4 583 538
- US-A- 4 608 977
- US-A- 4 638 798
- US-A- 4 645 343
- Robotics Age, vol.7, no.6, June 1985, Peterborough, New Hampshire (US) Y.S.Kwoh: "A new computerized tomographic-aided robotic stereotaxis system", pages 17-22
- "Neuronavigator", Hideyasu Watanabe, Zeitschrift "Igaku-no-Ayumi", Band 137, Nr. 6, 10.05.86, S. 451-452
- Polyscope plus, Ausgabe 6/86, S. 4-6, "Computer Aided Surgery, ein erster Schritt: Robotik für Hirnoperationen?", Dr. H.Reinhardt, H. Meyer, E. Amrein

## Beschreibung

Die Erfindung betrifft eine Vorrichtung zur Ausübung eines Verfahrens, bei welchem Schichtbilainformationen eines Körperteils in dem der chirurgische Eingriff durchzuführen ist, im Datenspeicher eines Datenverarbeitungssystems abgespeichert werden,
Lagedaten eines Eingriffsortes aus den Schichtbildinformationen bestimmt werden,
das chirurgische Instrument an einem dreidimensional frei beweglichen Träger angebracht wird,
Stellungsdaten des chirurgischen Instrumentes mit einer Koordinatenmeßvorrichtung bestimmt und dem Datenverarbeitungssystems zugeführt werden, das die Stellungsdaten des chirurgischen Instrumentes zu den Lagedaten des Eingriffsortes in Beziehung setzt, und
das chirurgische Instrument aufgrund dieser Beziehung zu dem Eingriffsort ausgerichtet wird, wobei
als Bezugspunkte angeordnet werden am Körperteil mindestens drei von außen zugängliche Meßpunkte bestimmt oder angeordnet stellt,
von dem Körperteil die Meßpunkte enthaltende Schichtaufnahmen angefertigt und im Datenspeicher abgelegt stellt,
mit der Koordinatenmeßvorrichtung die räumliche Lage der Meßpunkte erfaßt und die Meßdaten im Datenspeicher abgelegt, stellt
das Datenverarbeitungssystem eine Beziehung zwischen den in den Schichtaufnahmen enthaltenen Bilddaten der Meßpunkte und den durch die Koordinatenmeßvorrichtung ermittelten Daten der Meßpunkte her stellt,
mit der Koordinatenmeßvorrichtung die räumliche Stellung des dreidimensional frei beweglichen chirurgischen Instrumentes innerhalb des Körperteiles fortlaufend erfaßt wird und die Stellungsdaten dem Datenverarbeitungssystem zugeführt werden, wobei
das Datenverarbeitungssystem den Bildinformationen der Schichtaufnahmen die Stellungsdaten des chirurgischen Instrumentes überlagert und
das Datenverarbeitungssystem auf einem Ausgabegerät, insbesondere einem Bildschirm, Überlagerungsbilder erzeugt, bei denen die Bildinhalte der Schichtaufnahmen und die jeweiligen Stellungen des chirurgischen Instruments innerhalb des Körperteiles einander überlagert werden.

Bei vielen Operationen, insbesondere im, Kopfbereich, können Probleme bezüglich der Orientierung während der Operation aufgrund der individuellen Anatomievarianten auftreten. Es gibt zahlreiche Eingriffe, bei denen ein erhöhtes Risiko allein wegen des Problemes einer umfassenden exakten Orientierung während des Eingriffs festzustellen ist.

Eine fortlaufende Information über die Lage von Operationsinstrumenten im jeweiligen Körperteil, insbesondere die Kenntnis über den räumlichen Abstand zu verletzlichen Strukturen, wie beispielsweise Blutgefäßen, Lymphgefaßen, Nerven etc. erhöht die Sicherheit beim Operieren. Sofern diese Informationen festgehalten bzw. gespeichert werden könnten und damit reproduzierbar wären, ließe sich nach erfolgter Operation deren Ergebnis überprüfen. Im Falle eines unverschuldeten Mißerfolgs ließen sich auf diese Weise unberechtigte Schadensersatzansprüche wirksam abwehren.

Um sich bei Operationen im menschlichen Körper orientieren zu können, werden bislang herkömmliche Röntgenaufnahmen, Computertomographie-Aufnahmen und/oder in Ausnahmefällen auch intraoperative Durchleuchtungen ver- bzw. angewandt.

In den Röntgenaufnahmen werden primär knöcherne Strukturen dargestellt. Es ist daher üblich, sich die höher verdichtete Information von Computertomogrammen zur Operationsplanung zunutze zu machen. Die Umsetzung des Röntgenbefundes in operatives Vorgehen geschieht durch den Operateur. Dieser überprüft intraoperativ die exakte Lage des Operationsinstrumentes visuell. Gelegentlich wird das Operationsgebiet auch ausgemessen oder durchleuchtet. Letzteres ist mit allen Nachteilen konventioneller Röntgentechnik und höherer Strahlenbelastung für Patient und Operateur verbunden. Als weiterer schwerwiegender Nachteil bleibt festzuhalten, daß bei einer intraoperativen Seitdurchleuchtung bei der dadurch erhältlichen Abbildung die räumlichen Verhältnisse in dem zu operierenden Körperbereich nur überlagert dargestellt werden können. Es bedarf einer äußerst umfangreichen Erfahrung, um daraus zumindest annähernd exakte Rückschlüsse auf die tatsächlichen räumlichen Gegebenheiten anzustellen.

Eine fortlaufende sichere Information über die Lage des Operationsinstrumentes in bezug zum Krankheitsherd ist aber damit jedoch nicht möglich.

Alternativ zu den konventionellen Methoden besteht heute die Möglichkeit auf rechnerunterstützte Lageinformationen zurückzugreifen.

Im Bereich der Neurochirugie weden stereotaktische Operationen mit Hilfe eines Lokalisationsrahmens und eines Instrumentenhalters ausgeführt.

Ein stereotaktisches Chirurgiesystem und ein damit durchführbares Operationsverfahren der eingangs genannten Art sind in der DE-A-3 205 085 beschrieben. Danach wird zunächst bei einer definierten Lage des Patienten, die durch eine besondere Halterung sichergestellt wird, ein Röntgentomogramm des interessierenden Körperteils bzw. dessen Operationsbereiches aufgenommen. Dann wird in der betreffenden Schichtbildaufnahme der Operationsort aufgesucht, dessen Lagekoordinaten in Bezug auf den Patienten bzw. dessen Körperteil im Röntgentomogramm bestimmt sind. Ein starr mit dem Operationstisch gekoppeltes chirurgisches Instrument hat eine Spitze in einer Lage, die durch entsprechende Lagekoordinaten bestimmt ist. Die Beziehung zwischen den Lagekoordinaten des Operationsortes und den Lagekoordinaten dieser Spitze ergibt dann einen Operationsweg, entlang dessen der Operateur mit dem chirurgischen Instrument zum Operationsort gelangt. Dazu ist das chirurgische Instrument an einer Halterung angeordnet, die seine Verstellung in X-, Y- und Z-Richtung und zusätzlich entlang vorgegebener Kreisbögen in der XZ- und in der YZ-Ebene ermöglicht.

Eine weitere Anordnung und ein weiteres Verfahren dieser Art sind in der Veröffentlichung von Y. S. Kwoh et al. in der Zeitschrift "Robotics Age", Bd. 7, Nr. 6, Seiten 17 bis 22, unter dem Titel "A new computerized tomographic-aided robotic stereotaxis system" beschrieben. Auch bei dieser Anordnung ist eine starre Kopplung zwischen dem Tomographie- und dem Operationssystem und zu dem Patienten vorgesehen, wobei der zu untersuchende und zu operierende Körperteil des Patienten festgehalten wird. Im einzelnen ist zur Durchführung der Operation ein Roboter mit einem Gelenkarm vorgesehen, der das chirurgische Instrument trägt. Dieser Gelenkarm ist dreidimensional, d.h. um insgesamt 6 Drehachsen beweglich ist, aus deren Drehwinkeln die Lagekoordinaten des chirurgischen Instrumentes bestimmt werden. Es ist weiter ein Rechner vorgesehen, der sowohl die Daten des Tomogramms, also die Schichtbildinformationen als auch die Daten von der Robotersteuerung erhält und so miteinander verbindet, daß nach Bestimmung eines Eintrittspunktes für das chirurgische Instrument der Operationsweg festgelegt wird.

Weitere solche Verfahren und stereotaktischen Vorrichtungen sind auch aus den US-A-4 465 069, EP-A-0 207 452, EP-A-0 018 166 und DE-A-3 205 915 bekannt. Ein spezifischer V-förmiger Rahmen ist auch aus der US-A-4 583 538 bekannt geworden, der aber nicht auf dem Gebiet der Neurochirurgie, sondern für entprechende Operationen im Brutskorbbereich ausgebildet und angepaßt ist.

Die stereoteaktische Chirurgie ist ein Teilgebiet der Neurochirurgie und betrifft eine Klasse von Operationen, bei welchen Sonden, wie beispielsweise Kanülen, Nadel, Klemmen oder Elektroden an Gehirnstellen oder anderen verdeckten anatomischen Zielen angebracht werden sollen, die von außen her nicht sichtbar sind. Der stereotaktische Rahmen dient dabei als eine Art "Führungsvorrichtung", die in der Human-Neurochirurgie verwandt wird, um ein Instrument zu einem speziellen Punkt innerhalb des Gehirns durch eine kleine Öffnung in der Schädeldecke mit Hilfe radiographischer oder anderer Sichtbarmachung von Bezugspunkten zu führen. Dabei soll die Hinführung des Instrumentes an einen exakten vorbestimmten Punkt so genau als möglich erfolgen. Wenn man also den Rahmen oder die Vorrichtung an der Schädeldecke anbringt, dann kann man die Sonde zu einem gegebenen topographischen Punkt innerhalb des Schädels vorwärts bewegen. Der exakte Punkt wird dann aus der ermittelten Distanz und der Richtung zwischen dem wahrgenommenen Bezugspunkt und dem gewünschten Ziel in Bezug auf das Koordinatensystem der stereotaktischen Vorrichtung errechnet. Durch lineares Vorschieben des Instrumentes, welches über dem im Rahmen gehaltenen Instrumentenhalter exakt ausgerichtet ist, wird dann an dem gewünschten Punkt eine Probe entnommen, eine lokale Läsion gesetzt oder Strahlungsmaterial implantiert.

Derartige Methoden wurden weiterentwickelt, um sie weitgehend zu automatisieren oder um beispielsweise einen Laserkoagulator zu verwenden. Nach einem aus CT-Aufnahmen gewonnenen Plan können punktförmige Läsionen gesetzt werden. Diese bekannten Verfahren und Vorrichtungen bedürfen weiterhin des Einsatzes eines am Kopf fest justierten Rahmens. Dabei muß ferner bedacht werden, daß ein exakter Sitz des Rahmens nur dadurch erzielbar ist, daß zumindest drei Schrauben bis in den Schädelknochen fest eingedreht werden.

Aus der Zeitschriften-Veröffentlichung "Journal of Neurosurgery, Volume 65, October 1986, Seite 445 ff." ist auch eine berührungsfreie, d. h. rahmenlose Messung zur Erzielung einer rechnerunterstützten Lageinformation eines Instrumentes bekannt. Bei diesem Verfahren wird über drei akustische Signalgeber mittels Funkenstrecken und insgesamt vier Empfängern die exakte Lage eines Operationsmikroskopes festgestellt. Darüber können dann die vorher abgespeicherten Computertomographieaufnahmen auf die Fokusebene des Operationsmikroskopes projiziert werden, um eine entsprechende Hilfestellung während der Operation zu geben.

Aber auch bei diesem Verfahren handelt es sich grundsätzlich - wie in der Vorveröffentlichung auch erwähnt wird - um ein stereotaktisches Chirurgiesystem, das nur punktuell arbeitet, bei dem der Arbeitspunkt linear angefahren wird, und daß zudem im wesentlichen nur im Bereich des Hirnschädels und nicht jedoch im knöchernen Schädel eingesetzt wird. Dies mag auch darin begründet liegen, daß die angegebene Genauigkeit von über 2 mm unzureichend ist.

Schließlich ist aus der Veröffentlichung der medizinischen Fakultät der Universität Tokio (Igaku no Ayami 137(6), 10 Mai 1986) ein sogenannter Neuronavigator bekannt, der eine an einem dreidimensional bewegbaren Tragarn befestigte Armspitze aufweist, die sich bei ihrem Aufsetzen auf ein Körperteil verkürzt. Dabei wird entsprechend dem Ausmaß der Verkürzung anhand einer Mehrzahl von CT-Aufnahmen ein im Innenraum des Körperteils liegender Tumor auf die Körperoberfläche projiziert, um hindurch den Ort des späteren Operationsgeschehens zu bestimmen.

Bei allen bekannten Verfahren besteht ferner keine Möglichkeit bzw. ist es nicht beabsichtigt, den Ablauf und das Ergebnis des chirurgischen Eingriffs für eine nachträgliche Überprüfung bildlich zu dokumentieren.

Aufgabe der vorliegenden Erfindung ist es daher, eine Vorrichtung zu schaffen, die erstmals eine reproduzierbare Darstellung der vorher gewonnenen schichtaufnahmen zusammen mit fortlaufender Darstellung der Lage eines freihändig geführten Operationsinstrumentes in einem dreidimensionalen Modell des Körperteils auf dem Bildschirm erlaubt.

Schließlich ist aus der Veröffentlichung der medizinischen Fakultät der Universität Tokio (Hideyasu Watanabe: "Neuronavigator"; in der Zeitschrift Igaku-no-Ayumi, Band 137, Nr.6, 10.Mai 1986, Seiten 451,452) ein sogenannter Neuronavigator bekannt, der eine an einem dreidimensional bewegbaren Tragarm befestigte Armspitze aufweist, die sich bei ihrem Aufsetzen auf ein Körperteil verkürzt. Dabei wird entsprechend dem Ausmaß der Verkürzung anhand einer Mehrzahl von CT-Aufnahmen ein im Innenraum des Körperteils liegender Tumor auf die Körperoberfläche projiziert, um hindurch den Ort des späteren Operationsgeschehens zu bestimmen.

Vorteilhafte Ausgestaltungen der Erfindung sind in den Unteransprüchen angegeben.

Durch die vorliegende Erfindung werden gegenüber dem Stand der Technik wesentliche Vorteile erzielt. Erstmals ist es möglich, ständig auf einem Visualisierungssystem das Operationsgebiet und seine Umgebung in Form von vom Operateur frei wählbaren Schnitten darzustellen, weobei gleichzeitig fortlaufend die Position des Operations-Instrumentes in die Darstellung des Operationsgebiees miteingeblendet wird, wodurch sogenannte Überiagerungsbilder entstehen.

Da die Position des chirurgischen Instrumentes ständig aus den von der Koordinatenmeßvorrichtung der Führungsvorrichtung bestimmten Stellungsdaten berechnet wird, ist dadurch ein erneutes Röntgen auch während der Operation überflüssig. Die Röntgendosen werden auch dadurch gegenüber dem Stand der Technik (intraoperative Seitdurchleuchtung) verringert. Es können aber auch andere Schichtbild-Verfahren, wie beispielsweise die Kernspintomographie angewandt werden. Besonders vorteilhaft läßt sich das erfindungsgemäße Verfahren bzw. die erfindungsgemäße Vorrichtung im Bereich des Gesichtsschädels (aber nicht nur dort) einsetzen.

Eine Dokumentation der reproduzierbaren Bilddarstellungen kann dadurch erfolgen, daß fortlaufend fotografische Bilder von den auf dem Ausgabegerät sichtbaren Überlagerungsbildern angefertigt werden. Eine andere zweckmäßigere Möglichkeit der Dokumentation erfolgt in der Weise, daß die Bilddaten der Überlagerungsbilder in einem Datenspeicher des Datenverarbeitungssystems abgelegt werden, aus dem sie jederzeit wieder abrufbar und erneut auf dem Ausgabegerät darstellbar sind.

Die Abbildung des chirurgischen Instrumentes kann in der Weise erfolgen, daß die Lage der Spitze des Instrumentes als ein Punkt oder Fadenkreuz auf dem Bildschirm angezeigt wird. Diese Darstellungsart ist für die Durchführung des chirurgischen Eingriffes völlig ausreichend, weil der Chirurg das chirurgische Instrument manuell führt und dabei dessen räumliche Längserstreckung bestimmt. Für die Dokumentation des chirurgischen Eingriffes und nachträgliche Überprüfbarkeit des Operationsablaufes kann es dagegen zweckmäßig sein, wenn nicht nur die Spitze sondern zumindest ein Teil des gesamten Instrumentes abgebildet wird, damit auf dem Überlagerungsbild die Längserstreckung und die momentane Ausrichtung des chirurgischen Instrumentes erkennbar sind.

Weitere Vorteile, Einzelheiten und Merkmale der Erfindung ergeben sich nachfolgend anhand zweier in den Zeichnungen dargestellter Ausführungsbeispiele. Dabei zeigen im einzelnen:
- Figur 1:: eine schematische Darstellung zur Aufnahme einer Computertomographie;
- Figur 2 :: die anhand der Computertomographie hergestellten Schichtbildaufnahmen;
- Figur 3 :: eine vereinfachte schaubildliche Darstellung einer Koordinatenmeßvorrichtung mit eingesetztem chirurgischem Instrument in Verbindung mit dem zu behandelnden Körperteil eines Patienten;
- Figur 4 :: einen Computer mit Graphikbildschirm;
- Figur 5 :: eine erfindungsgemäß aufgearbeitete überlagernde Darstellung des chirurgischen Instrumentes mit einem Schichtbild;
- Figur 6 :: eine schaubildliche Darstellung der Koordinatenmeßvorrichtung;
- Figur 7 :: eine Schnittdarstellung einer mit einem Codeabtaster ausgestatteten Kupplung zur Aufnahme eines mit Codezeichen versehenen Instrumententrägers;
- Figur 8 :: eine Seitenansicht des Instrumententrägers mit Codezeichen;
- Figur 9 :: eine Ansicht eines teilweise dargestellten Gelenkarmes einer zweiten Ausführungsform einer Koordinatenmeßvorrichtung mit einem Gewichtsausgleichsmechanismus;
- Figur 10:: eine teilweise geschnittene Draufsicht auf den in gestreckter Lage dargestellten Gelenkarm nach Figur 9.

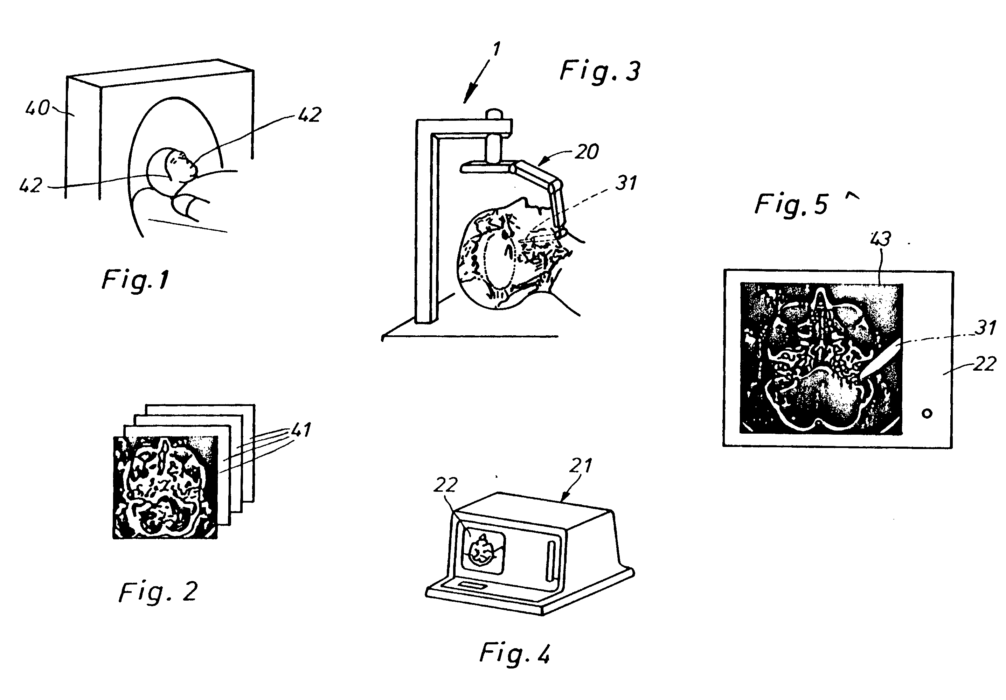

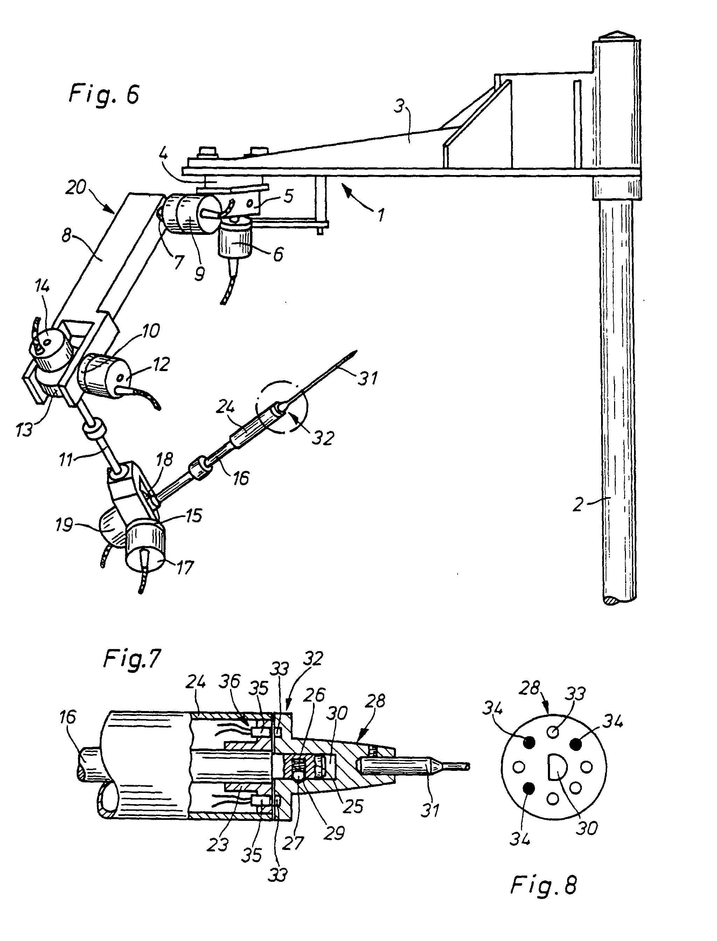

In Figur 3 ist eine vereinfacht dargestellte Koordinatenmeßvorrichtung (1) gezeigt, deren konstruktiver Aufbau aus Figur 6 hervorgeht. Die Koordinatenmeßvorrichtung (1) hat einen auf einer Stange (2) einstellbaren Ausleger (3). Der Ausleger (3) ist über ein Gelenk (4) mit einem in horizontaler Ebene verschwenkbaren Arm (5) verbunden. Am Gelenk (4) ist ein Drehmelder (6) angeordnet, der ein der jeweiligen Winkelstellung des Armes (5) entsprechendes Signal abgibt.

Am Arm (5) ist über ein Gelenk (7) ein in vertikaler Ebene schwenkbarer Arm (8) gelagert, dessen Winkelstellung durch einen Drehmelder (9) erfaßt wird. Am anderen gabelförmig ausgebildeten Ende des Armes (8) ist mittels eines Gelenkes (10) eine Trägerstange (11) schwenkbar angeordnet, deren Schwenklage durch einen Drehmelder (12) erfaßt wird. Die Trägerstange (11) ist ferner über ein Gelenk (13) um ihre Längsachse drehbar. Die Drehlage der Trägerstange (11) wird durch einen Drehmelder (14) erfaßt.

Am anderen gabelförmig ausgebildeten Ende der Trägerstange (11) ist mittels eines Gelenkes (15) eine zweite Trägerstange (16) schwenkbar angeordnet, deren Schwenklage durch einen Drehmelder (17) erfaßt wird. Die Trägerstange (16) ist ferner über ein Gelenk (18) um ihre Längsachse drehbar. Die Drehlage der Trägerstange (16) wird durch einen Drehmelder (19) erfaßt.

Die Bauelemente (3) bis (19) bilden einen Gelenkarm (20) mit sechs Drehachsen.

Die Gelenke (4), (7), (10), (13), (15), (18) sind so ausgebildet, daß sie selbsthemmend sind, jedoch mit geringem Kraftaufwand bewegt werden können. Bei den Drehmeldern (6), (9), (12), (14), (17), (19) handelt es sich um inkrementale Drehmelder mit 4.000 Impulsen pro Umdrehung. Aufgrund dieser großen Impulszahl ergibt sich eine sehr genaue Winkelerfassung der Schwenk- und/oder Drehstellung der betreffenden Glieder des Gelenkarmes (20). Die Drehmelder (6), (9), (12), (14), (17), (19) sind über nicht bezeichnete Kabel mit einem in Figur 4 schaubildlich dargestellten Datenverarbeitungssystem (21) verbunden, das einen Computer, einen Datenspeicher und als Ausgabegerät einen Bildschirm (22) umfaßt.

Auf der Trägerstange (16) ist im Bereich ihres freien Endes ein Flansch (23) befestigt, dessen Umfangsseite durch eine Hülse (24) abgedeckt ist. An der Trägerstange (16) ist im Anschluß an den Flansch (23) ein einseitig abgeflachter Zapfen (25) ausgebildet. In einer quer verlaufenden Sackbohrung (26) des Zapfens (25) ist eine federbelastete Kugel (27) angeordnet, die durch eine entsprechende nicht dargestellte Verengung am Rand der Bohrung (26) gegen Herausfallen gesichert ist.

Auf dem Zapfen (25) ist ein Instrumententräger (28) aufgesteckt, der eine der Kugel (27) zugeordnete und mit dieser ein Rastgesperre bildende Vertiefung (29) aufweist. Der Träger (28) enthält eine der Form des abgeflachten Zapfens (25) entsprechend ausgebildete Bohrung (30), wodurch der Träger (28) gegen Verdrehen gesichert ist. Im Träger (28) ist ein chirurgisches Instrument (31) lösbar befestigt. Somit bilden der Träger (28) und der Zapfen (25) eine Kupplung (32) zum Verbinden des chirurgischen Instrumentes (31) mit dem Gelenkarm (20) der Koordinatenmeßvorrichtung (1), die somit zugleich eine Führungs- und Haltevorrichtung für das Instrument (31) bildet.

An der Stirnfläche eines Flanschteiles des Instrumententrägers (28) sind mehrere kreisförmig angeordnete Sackbohrungen (33) vorgesehen, in denen drei Permanentmagnete (34) befestigt sind. Die Anordnung der Permanentmagnete (34) bildet einen dem eingesetzten Instrument (31) zugeordneten Kennzeichencode.

Im Flansch (23) sind eine der Anzahl der Sackbohrungen (33) entsprechende Anzahl von Hallgeneratoren (35) angeordnet, die den Bohrungen (33) bzw. den in diesen eingesetzten Permanentmagneten (34) genau gegenüberliegen und einen Codeabtaster (36) bilden. Die Permanentmagnete (34) bewirken, daß die ihnen zugeordneten Hallgeneratoren (35) ein Signal erzeugen. Die von den Hallgeneratoren (35) bzw. dem Codeabtaster (36) erzeugten Signale werden dem Datenverarbeitungssystem (21) zugeführt, wodurch dieses über die Art und Größe des mit dem Gelenkarm (20) verbundenen chirurgischen Instrumentes (31) informiert wird.

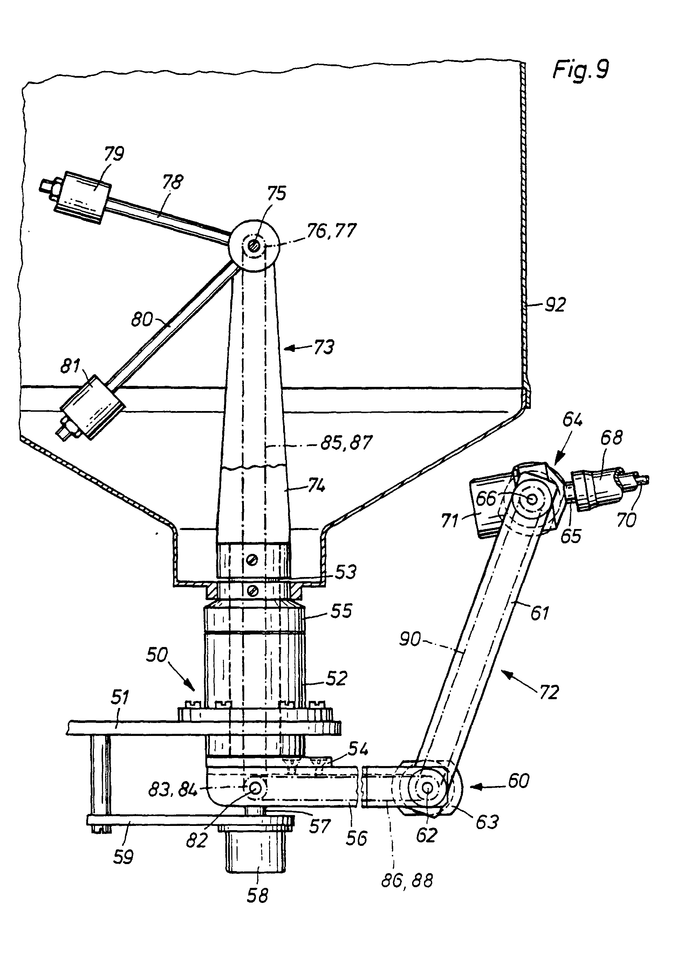

Die in Fig. 9 teilweise dargestellte Koordinatenmeßvorrichtung (50) weist einen an einer nicht dargestellten Stange einstellbar angeordneten Ausleger (51) auf. Auf dem Ausleger (51) ist ein flanschartiges Gelenkteil (52) befestigt, in dem ein hohler Gelenkbolzen (53) drehbar gelagert ist. Am unteren Ende des Gelenkbolzens (53) ist eine quer abstehende Trägerplatte (54) ausgebildet. Auf einem über das Gelenkteil (52) hinausragenden Abschnitt des Gelenkbolzens (53) ist ein Stellring (55) festgeklemmt, der gemeinsam mit der Trägerplatte (54) den Gelenkbolzen (53) axial sichert.

An der Trägerplatte (54) ist ein quer abstehender hohler Arm (56) befestigt. Am Arm (56) ist ein koaxial zum Gelenkbolzen (53) verlaufender Zapfen (57) befestigt, der die Drehstellung des Armes (56) auf einen Drehmelder (58) überträgt, welcher über eine Halterung (59) an dem Ausleger (51) verdrehsicher angeordnet ist.

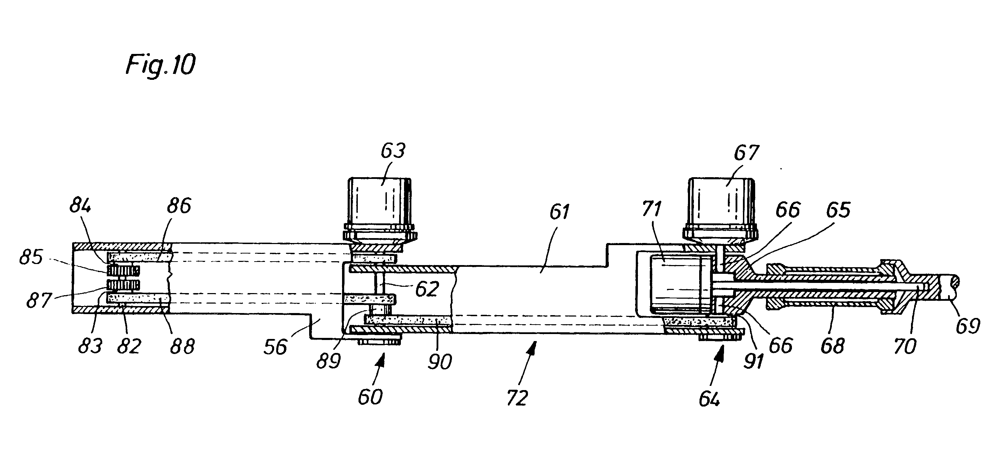

Am Arm (56) ist über ein Gelenk (60) ein in vertikaler Ebene schwenkbarer, hohl ausgebildeter Arm (61) gelagert, dessen Winkelstellung über einen Gelenkbolzen (62) auf einen mit dem Arm (56) verbundenen Drehmelder (63) übertragen wird. Am anderen Ende des Armes (61) ist mittels eines Gelenkes (64) eine hohl ausgebildete Trägerstange (65) schwenkbar gelagert, deren Schwenklage über einen von zwei miteinander fluchtenden Gelenkbolzen (66) (Fig. 10) auf einen am Arm (61) angeordneten Drehmelder (67) übertragen wird. Auf der Trägerstange (65) ist eine Hülse (68) drehbar gelagert, die mit einer zweiten Trägerstange (69) (Fig. 10) drehfest verbunden ist. Die Drehlage der zweiten Trägerstange (69) gegenüber der ersten Trägerstange (65) wird über eine Welle (70) auf einen an der Trägerstange (65) angeordneten Drehmelder (71) übertragen.

Die Bauelemente (51 bis 71) sind Bestandteile eines Gelenkarmes (72), der ähnlich wie der Gelenkarm (20) (Fig. 6) aufgebaut ist und somit außer diesen Bauelementen noch eine weitere nicht dargestellte quer und längs drehbare Trägerstange aufweist. Der Gelenkarm (72) hat auf diese Weise wie der Gelenkarm (20) sechs Drehachsen. An der nicht dargestellten Trägerstange ist über eine der Kupplung (32) entsprechende nicht dargestellte Kupplung ein gleichfalls nicht dargestellter Instrumententräger angeschlosen, der wie der Instrumententräger (28) aufgebaut ist.

Der Gelenkarm (72) ist mit einem Gewichtsausgleichsmechanismus (73) versehen. Dieser weist einen auf dem oberen Ende des Gelenkbolzens (53) aufgeklemmten, gabelförmig ausgebildeten Träger (74) auf, der an seinem oberen Ende einen feststehenden Bolzen (75) enthält. Auf dem Bolzen (75) sind zwei Zahnriemenräder (76, 77) unabhängig voneinander frei drehbar gelagert. Mit dem vorderen Zahnriemenrad (76) ist eine Stange (78) fest verbunden, an deren Ende eine Ausgleichsmasse (79) angeordnet ist. Mit dem hinteren Zahnriemenrad (77) ist eine längere Stange (80) fest verbunden, an deren Ende eine Ausgleichsmasse (81) angeordnet ist.

Im Arm (56) ist ein parallel zum Bolzen (75) verlaufender Bolzen (82) fest angeordnet, auf dem zwei unabhängig voneinander frei drehbare Doppelzahnriemenräder (83, 84) gelagert sind. Das hintere Zahnriemenrad (77) und das innere Rad des hinteren Doppelzahnriemenrades (84) sind durch einen Zahnriemen (85) miteinander verbunden. Das äußere Rad des hinteren Doppelzahnriemenrades (84) ist über einen Zahnriemen (86) mit einem nicht dargestellten Zahnriemenrad verbunden, das auf dem Gelenkbolzen (62) angeordnet und mit dem Arm (61) drehfest verbunden ist. Aufgrund dieser Konstruktion werden Schwenkbewegungen des Armes (61) über die beiden Zahnriemen (86 und 85) auf das hintere Zahnriemenrad (77) übertragen, wodurch die Stange (80) mit der Ausgleichsmasse (81) im gleichen Drehsinn verschwenkt wird. Bei sich horizontal erstreckendem Arm (61) nimmt die Stange (80) eine im wesentlichen ebenfalls horizontale Lage ein und bei sich vertikal erstreckendem Arm (61) eine im wesentlichen ebenfalls vertikale Lage. Dabei sind die Ausrichtlagen von Arm (61) und Stange (80) einander entgegengesetzt, d. h., daß sich die Stange (80) in abgesenkter Stellung befindet, wenn der Arm (61) angehoben ist und umgekehrt. Bei einer die Gewichtskraft des Armes (61) und der von ihm getragenen weiteren Bauelemente des Gelenkarmes (72) berücksichtigenden Bemessung der Ausgleichsmasse (81) wird von dieser ein Drehmoment erzeugt, das dem von der wirksamen Gewichtskraft des Armes (61) und den von diesem getragenen Bauelementen des Schwenkarmes (72) hervorgerufenen Drehmoment entgegengerichtet ist und aufgrund des Gewichtsausgleichs bewirkt, daß der Arm (61) in den beiden entgegengesetzten Schwenkrichtungen mit weitgehend gleichmäßiger geringer Kraft bewegt werden kann.

Das vordere Zahnriemenrad (76) und das innere Rad des vorderen Doppelzahnriemenrades (83) sind durch einen Zahnriemen (87) miteinander verbunden. Das äußere Rad des vorderen Doppelzahnriemenrades (83) ist über einen Zahnriemen (88) mit einem Rad eines Doppelzahnriemenrades (89) verbunden, das auf dem Gelenkbolzen (62) frei drehbar angeordnet ist. Das andere Rad des Doppelzahnriemenrads (89) ist über einen Zahnriemen (90) mit einem Zahnriemenrad (91) verbunden, das auf dem vorderen Gelenkbolzen (66) angeordnet und mit der Trägerstange (65) drehfest verbunden ist.

Durch die Zahnriemen (90, 88 und 87) werden die Schwenkbewegungen der Trägerstange (65) auf das vordere Zahnriemenrad (76) übertragen, wodurch die Stange (78) mit der Ausgleichsmasse (79) im gleichen Drehsinn verschwenkt wird. Hierbei sind die Bewegungsverhältnisse zwischen der Trägerstange (65) und der Ausgleichsmasse (79) vergleichbar den vorstehend beschriebenen Bewegungsverhältnissen zwischen dem Arm (61) und der Ausgleichmasse (81). Bei entsprechender Bemessung der Ausgleichsmasse (79) wird von dieser ein Drehmoment erzeugt, das dem von der wirksamen Gewichtskraft der Trägerstangen (65, 69), der Hülse (68) und den von der Trägerstange (69) getragenen Bauelementen des Gelenkarmes (72) hervorgerufenen Drehmoment entgegengerichtet ist. Aufgrund dieses Gewichtsausgleiches können die Trägerstangen (65, 69) und die Hülse (68) bei ihren Schwenkbewegungen um die Gelenkbolzen (66) mit weitgehend gleichmäßiger geringer Kraft bewegt werden.

Auf dem Stellring (55) ist ein zweiteiliges geschlossenes Gehäuse (92) aufgeklemmt, das die Bewegungsbahnen der Ausgleichsmassen (79, 81) umschließt.

In einer weiteren, nicht dargestellten Ausführungsform eines Gelenkarmes sind die sechs Drehmelder gemeinsam in einem dem Gehäuse (92) vergleichbaren Gehäuse untergebracht, wobei die einzelnen Drehmelder über jeweils eigene Zahnriemen- oder Zahnradgetriebe mit den einzelnen Gliedern des Gelenkarmes verbunden sind. Durch diese Maßnahme ist der Gelenkarm nicht nur masseärmer und dadurch leichter handhabbar, sondern er ist auch schlanker wodurch die Kollisionsgefahr verringert wird.

Die Funktionsweise der Vorrichtung werden nachfolgend anhand eines typischen Ablaufes näher erläutert.

Zunächst wird eine zu behandelnde Person (wie in Figur 1 dargestellt) zur Aufnahme mehrerer Schichtbilder in ein geeignetes Schichtbild-Aufnahmegerät (40) geschoben. Beispielsweise können hier Computertomographie-Aufnahmen oder beispielsweise Kernspintomographien hergestellt werden.

In Figur 2 sind schematisch die entsprechend gemäß Figur 1 aufgenommenen Schichtaufnahmen (41) dargestellt.

Vor der Aufnahme sind in dem interessierenden Bereich einer zu operierenden Person drei Meßpunkte (42) markiert, befestigt, ausgemessen oder festgelegt, von denen zwei im Bereich der Ohren liegen, während der dritte beispielsweise durch den unten auslaufenden Spalt zwischen den beiden oberen Schneidezähnen gebildet sein kann.

Sofern aufgrund der tatsächlichen Verhältnisse nicht bestimmte Meßpunkte festgelegt werden können, so können auch beispielsweise kleine Keramikteilchen an vorbestimmten Stellen als Meßpunkt eingesetzt und angebracht werden. Keramikteilchen eignen sich insbesondere, weil diese bei den entsprechenden Aufnahmen keine Reflektion hervorrufen.

Die erwähnten Meßpunkte (42) sind auf den in Figur 2 wiedergegebenen Schichtaufnahmen (41) an der jeweils betreffenden Stelle bzw. Lage abgebildet und von den Daten her mitumfaßt. Die mit den Daten der Meßpunkte versehenen Schichtbilddaten, die in ihrer Gesamtheit eine räumliche Struktur des zu behandelnden Körperteils wiedergeben, werden in einem entsprechenden Speicher des Datenverarbeitungssystems abgespeichert.

Zur Vorbereitung einer durchzuführenden Operation wird der Patient auf einem Operationstisch liegend gehalten und justiert. Vor Durchführung der Operation wird zunächst über die erwähnte Koordinatenmeßvorrichtung (1) die Lage der drei am Patienten angebrachten und befestigten bzw. festgelegten Meßpunkte (42) festgestellt. Dies geschieht in der Weise, daß das chirurgische Instrument (31) oder ein stattdessen verwendeter Taster mit den Meßpunkten (42) in Berührung gebracht wird, wobei das Instrument oder der Taster aufgrund der Gelenkigkeit des Gelenkarmes (20) völlig unbehindert angehoben, abgesenkt, geneigt, winkelig verstellt und vorgeschoben werden kann. Jede Bewegung und Lage der einzelnen Glieder des Gelenkarmes (20) wird über die Drehmelder (6), (9), (12), (14), (17), (19) exakt erfaßt und dem Datenverarbeitungssystem (21) übermittelt. Die auf diese Weise gewonnenen Lagedaten der Meßpunkte (42) werden mit den Bilddaten der Meßpunkte (42) im Datenspeicher (7) überprüft. Durch entsprechende Berechnung des Computers werden die am Operationstisch in ihrer Lage festgestellten Meßpunkte (42) so in Übereinstimmung mit den abgespeicherten Bilddaten der Meßpunkte (42) gebracht, daß nunmehr eine exakte Zuordnung der abgespeicherten Schichtaufnahmedaten mit der konkreten räumlichen Lage des Patienten und v. a. des chirurgischen Instrumentes (31) vorgenommen wird.

Mit Hilfe der vom Codeabtaster (36) übermittelten Signale ruft der Computer dem entsprechenden chirurgischen Instrument (31) zugeordnete, im Datenspeicher abgelegte Kennzeichenwerte über die Größe und die Entfernung der Instrumentenspitze vom Instrumententräger (28) ab, wodurch bei jedem der verschiedenen Instrumente bei einer beliebigen Stellung des Instrumententrägers (28) durch den Computer die genaue Lage der Instrumentenspitze berechnet wird.

Nachdem über die Koordinatenmeßvorrichtung (1) die drei Meßpunkte (42) angefahren und dadurch die exakte, räumliche Zuordnung zwischen chirurgischem Instrument (31) und Patient in Übereinstimmung mit den gespeicherten Schichtaufnahmen (41) hergestellt worden ist, kann mit der Operation begonnen werden, so daß nunmehr bei einer Bewegung und einem entsprechenden Eingriff die Spitze bzw. der wirksame Bereich des chirurgischen Instrumentes über die Koordinatenmeßvorrichtung (1) bei jeder auch noch so kleinen Bewegung oder/und Winkelbewegung erfaßt und mittels des Computers festgestellt werden kann. Diese erfaßte Bewegung des chirurgischen Instrumentes wird dann über den Computer auf dem Bildschirm (22), gemeinsam mit der jeweils aktuellen Schichtbildaufnahme abgebildet, wodurch ein Überlagerungsbild (43) entsteht.

Die Bilddaten der Überlagerungsbilder (43) werden zur Dokumentation des Ablaufes des chirurgischen Eingriffes im Datenspeicher abgelegt, aus dem sie jederzeit wieder abrufbar und erneut auf dem Bildschirm (22) darstellbar sind.

Sobald sich bei jedem weiteren Verschieben, Eindringen oder Verdrehen des chirurgischen Instrumentes die Instrumentenspitze aus der momentan auf dem Bildschirm (22) dargestellten Schichtaufnahme herausbewegt, wird anstelle dieser Aufnahme automatisch diejenige Schichtaufnahme abgebildet, in die sich die Instrumentenspitze nunmehr hineinbewegt. Dadurch wird für einen Operateur die maximale Information darüber vermittelt, in welchem exakten Bereich er sich während der Operation befindet.

Eine weitere Maßnahme zur besseren Information über die räumliche Stellung der Instrumentenspitze innerhalb des Operationsgebietes läßt sich dadurch erreichen, daß in verschiedenen Fenstern des Bildschirmes (22) gleichzeitig Überlagerungsbilder dargestellt werden, die von längs, quer und horizontal verlaufenden Schichtaufnahmen gebildet wurden.

Die Hilfe durch die geschilderte Vorrichtung kann noch dadurch erhöht werden, daß beispielsweise je nach Bedarf eine der jeweils weiter tiefer liegenden nächsten Schichten vorab auf dem Bildschirm (22) dargestellt werden kann, um schon vorher zu überlegen, in welcher Richtung nachfolgend das chirurgische Instrument (31) weiter fortbewegt werden soll. Auch hierdurch wird die Sicherheit gegenüber herkömmlichen Verfahren und Vorrichtungen erheblich verbessert.

Die geschilderte Koordinatenmeßvorrichtung (1) dient also im vorliegenden Fall nicht nur zur Positionserfassung des Operations-Instrumententrägers (28) bzw. des Instrumentes (31) selbst, sondern auch zur Erfassung der drei Meßpunkte (42). Dies bietet den weiteren Vorteil, daß auch während der Operation jederzeit überprüft werden kann, ob die exakte Lage des Patienten beibehalten worden ist. Dazu müssen lediglich über ein Instrument die drei Meßpunkte (42) auch während der Operation abgefahren und die entsprechenden Lagedaten jeweils in den Computer eingegeben werden. Sollte eine leichte Lageveränderung des zu behandelnden Körperteiles festgestellt worden sein, so kann diese Lageveränderung sofort rechnerisch erfaßt und das auf dem Bildschirm (22) dargestellte Bild korrigiert werden.

Im gezeigten Ausführungsbeispiel ist das chirurgische Instrument der Koordinatenmeßvorrichtung (1) am Gelenkarm (20) lösbar angebracht bzw. mit diesem verbunden. Möglich ist aber genauso, daß das chirurgische Instrument kontaktlos über eine "Ortungsvorrichtung", also eine Koordinatenmeßvorrichtung ständig überwacht wird und dadurch die exakten Lagekoordinaten der Spitze bzw. des wirksamen Bereiches des Instrumentes festgestellt und an den Computer weitergeleitet werden. Dies kann durch beispielsweise drei räumlich angeordnete Sonden und drei Detektoren erfolgen.

Ob dabei die Lagekoordinaten-Feststellung aufgrund eines akustischen oder optischen oder elektromagnetischen Weges erfolgt (z. B. oberhalb oder unterhalb des Lichtwellenbereiches), hängt vom Einzelfall ab.

Werden mit dem chirurgischen Instrument bestimmte Körperteile entfernt, so können durch Abfahren der dabei entstandenen Höhlungen in Form einer Hüllkurve die so gewonnenen Daten während der Operation in den Datenspeicher eingegeben und dementsprechend die vor der Operation gewonnenen Schichtbilddaten modifiziert werden. Damit lassen sich auf dem Bildschirm (22) die aktuellen, während der Operation geänderten Verhältnisse darstellen. Die auf diese Weise gewonnenen postoperativen Schichtbilder werden ferner zur Dokumentation des Operationsergebnisses im Datenspeicher abgelegt.

## Patentansprüche

1. Vorrichtung zur Durchführung eines Verfahrens zur reproduzierbaren optischen Darstellung eines mit einem chirurgischen Instrument durchzuführenden Eingriffes bei welchem
Schichtbilainformationen eines Körperteils in dem der chirurgische Eingriff durchzuführen ist, im Datenspeicher eines Datenverarbeitungssystems (21) abgespeichert werden,
Lagedaten eines Eingriffsortes aus den Schichtbildinformationen bestimmt werden, das chirurgische Instrument (31) an einem dreidimensional frei beweglichen Träger (16) angebracht wird,
Stellungsdaten des chirurgischen Instrumentes (31) mit einer Koordinatenmeßvorrichtung (1;50) bestimmt und dem Datenverarbeitungssystems (21) zugeführt werden, das die Stellungsdaten des chirurgischen Instrumentes (31) zu den Lagedaten des Eingriffsortes in Beziehung setzt, und
das chirurgische Instrument (31) aufgrund dieser Beziehung zu dem Eingriffsort ausgerichtet wird, wobei
als Bezugspunkte am Körperteil mindestens drei von außen zugängliche Meßpunkte (42) bestimmt oder angeordnet, werden,
von dem Körperteil die Meßpunkte (42) enthaltende Schichtaufnahmen, (41) angefertigt und im Datenspeicher abgelegt, werden
mit der Koordinatenmeßvorrichtung (1;50) die räumliche Lage der Meßpunkte (42) erfaßt und die Meßdaten im Datenspeicher abgelegt, werden
das Datenverarbeitungssystem (21) eine Beziehung zwischen den in den Schichtaufnahmen (41) enthaltenen Bilddaten der Meßpunkte (42) und den durch die Koordinatenmeßvorrichtung (1;50) ermittelten Daten der Meßpunkte (42) her stellt,
mit der Koordinatenmeßvorrichtung (1;50) die räumliche Stellung des dreidimensional frei beweglichen chirurgischen Instrumentes (31) innerhalb des Körperteiles fortlaufend erfaßt wird und die Stellungsdaten dem Datenverarbeitungssystem (21) zugeführt werden, wobei
das Datenverarbeitungssystem (21) den Bildinformationen der Schichtaufnahmen (41) die Stellungsdaten des chirurgischen Instrumentes (31), überlagert und
das Datenverarbeitungssystem (21) auf einem Ausgabegerät, insbesondere einem Bildschirm (22), Überlagerungsbilder (43), erzeugt bei denen die Bildinhalte der Schichtaufnahmen (41) und die jeweiligen Stellungen des chirurgischen Instruments (31) innerhalb des Körperteiles einander überlagert werden,
ein Datenverarbeitungssystem (21);
einen Datenspeicher in dem Datenverarbeitungssystem (21), in dem Schichtbildinformationen eines Körperteils in Form von einer Reihe von Schichtbildaufnahmen (41) gespeichert sind;
einen mit dem Datenverarbeitungssystem (21) verbundenen Bildschirm (22) zur Wiedergabe ausgewählter Schichtbildaufnahmen (41) aus den Schichtbildinformationen;
einen dreidimensional frei beweglichen Träger (16), der ein chirurgisches Instrument (31) trägt;
eine mit dem Träger (16) und dem Datenverarbeitungssystem (21) gekoppelte Koordinatenmeßvorrichtung (1;50) zur Bestimmung der Stellungsdaten des chirurgischen Instrumentes (31), die durch das Datenverarbeitungssystem (21) zu Lagedaten in den Schichtbildinformationen in Beziehung setzbar sind;
mindestens drei an dem Körperteil bestimmte, von außen zugängliche Messpunkte (42);
Schichtbildinformationen in denen die Messpunkte (42) enthalten sind;
Mittel im Datenverarbeitungssystem (21) zur Herstellung einer Beziehung zwischen den von der Koordinatenmeßvorrichtung (1;50) abgetasteten Lagedaten der Messpunkte (42) in den Schichtbildinformationen und damit allgemein zwischen Lagedaten der Schichtbildinformationen und den durch die Koordinatenmeßvorrichtung (1;50) ermittelten Stellungsdaten des chirurgischen Instrumentes (31);
Mittel in dem Datenverarbeitungssystem (21) zur Überlagerung der während eines chirurgischen Eingriffes in den Körperteil aufgrund der besagten Beziehung ermittelten Stellungsdaten des chirurgischen Instrumentes mit den Schichtbildinformationen und zur Erzeugung von Überlagerungsbildern (43);
mit dem Datenverarbeitungssystem (21) verbundene Mittel zur fortlaufenden Wiedergabe von Überlagerungsbildern (43) auf dem Bildschirm (22) welche fortlaufend die Stellungen des chirurgischen Instrumentes in den Schichtbildaufnahmen (41) anzeigen;
mit dem Datenverarbeitungssystem (21) verbundene Mittel um auf dem Bildschirm (22) diejenige Schichtbildaufnahme (41) in der sich das chirurgische Instrument (31) momentan befindet, dann gegen die ihr benachbarte Schichtbildaufnahme (41) auszutauschen, wenn sich das chirurgische Instrument (31) vom Bereich dieser Schichtbildaufnahme (41) in den Bereich der benachbarten Schichtbildaufnahme (41) bewegt hat.
Mittel im Datenverarbeitungssystem (21) um die in zeitlichen Abständen mittels der mittels der Koordinatenmeßvorrichtung (1;50) ermittelten momentanen Lagedaten der Meßpunkte (42) mit den ursprünglichen Lagedaten der Meßpunkte (42) zu vergleichen und bei Abweichungen eine Lagekorrektur der auf dem Ausgabegerät dargestellten Schichtbildaufnahme (41) durchzuführen.

2. Vorrichtung nach Anspruch 1, **dadurch gekennzeichnet, daß** der Träger (16) für das chirurgische Instrument (31) an einem dreidimensional frei beweglichen Gelenkarm (20; 72) angebracht ist und die Koordinatenmeßvorrichtung (1; 50) mit dem Gelenkarm (20;72) verbundene Drehmelder aufweist.

3. Vorrichtung nach Anspruch 1, **dadurch gekennzeichnet, daß** die Koordinatenmeßvorrichtung als eine Ortungsvorrichtung mit kontaktloser Bestimmung der Stellungsdaten des chirurgischen Instrumentes (31) ausgebildet ist.

4. Vorrichtung nach Anspruch 2, **dadurch gekennzeichnet, daß** der Gelenkarm (20; 72) sechs Drehachsen enthält.

5. Vorrichtung nach Anspuch 4, **dadurch gekennzeichnet, daß** der Gelenkarm (72) einen Gewichtsausgleichmechanismus (73) aufweist.

6. Vorrichtung nach Anspruch 5, **dadurch gekennzeichnet, daß** zwei um horizontale Achsen (62; 66) schwenkbare Glieder (61; 65, 68, 69) des Gelenkarmes (72) über Zahnriemengetriebe (85, 86; 87, 88, 90) mit je einer eigenen Ausgleichsmasse (79, 81) verbunden sind.

7. Vorrichtung nach einem oder mehreren der Ansprüche 1 bis 6, **dadurch gekennzeichnet, daß** die chirurgischen Instrumente (31) und/oder ein Taster über eine Kupplung (32) mit der Koordinatenmeßvorrichtung (1;50) verbindbar und mit einem Kennzeichencode (34) versehen sind, und daß im Bereich der Kupplung (32) ein Codeabtaster (36) angeordnet ist, der zur Weitergabe der Codeinformation mit dem Datenverarbeitungssystem (21) verbunden ist.

8. Vorrichtung nach einen oder mehreren der Ansprüche 1 und 4 bis 7 **dadurch gekennzeichnet, daß** die den einzelnen Gelenken zugeordneten Meßgeräte der Koordinatenmeßvorrichtung (1;50) in einem zentralen Gehäuse angeordnet und durch Zugmittel- und Zahnradgetriebe mit den Gelenken verbunden sind.

## Claims

1. Device for carrying out a technique for reproducible optical representation of an operation carried out with a surgical instrument (31) in which operation
tomographic information of a body part in which the surgical operation is to be carried out is stored in the data memory of a data processing system (21),
positional data of an operating site are determined by the tomographic information,
the surgical instrument (31) is mounted on a three-dimensionally freely movable carrier (16),
attitude data of the surgical instrument (31) are determined by a co-ordinate measuring device (1;50) and fed to the data processing system (21) which relates the attitude data of the surgical instrument (31) to the positional data of the operation site, and
the surgical instrument (31) is aligned with respect to the operating site on the basis of this relationship,
wherein
at least three measurement points (42) accessible from outside are determined or arranged as reference points on the body part,
tomograms (41) of the body part which contain the measurement points (42) are produced and filed in the data memory,
the spatial position of the measurement points (42) is measured by the co-ordinate measuring device (1;50) and the measurement data are filed in the data memory,
the data processing system (21) produces a relationship between the image data contained in the tomograms (41) of the measurement points (42) and the data determined by the co-ordinate measuring device (1;50) of the measurement points (42),
the spatial attitude of the three-dimensionally freely movable surgical instrument (31) is continuously measured within the body part with the aid of the co-ordinate measuring device (1;50) and the attitude data are fed to the data processing system (21), and
the data processing system (21) superimposes the attitude data of the surgical instrument (31) on the image information of the tomograms and
the data processing system (21) generates on an output device, in particular a display screen (22), superimposition images (43) in which the image contents of the tomograms (41) and the respective attitudes of the surgical instrument (31) are superimposed on one another,
the device includes:
a data processing system (21),
a data memory in the data processing system (21) in which tomogram information of a body part in the form of a series of tomograms (41) is stored,
a display screen (22) connected with the data processing system (21) for displaying selected tomograms (41) of the tomogram information,
a three-dimensionally freely movable carrier (16) which carries a surgical instrument (31),
a co-ordinate measuring device (1;50) coupled with the carrier (16) and the data processing system (21) for determining the attitude data of the surgical instrument (31) which data are relatable to attitude data in the tomogram information by means of the data processing system (21),
at least three determined measurement points (42) on the body accessible from outside,
tomogram information in which the measurement points (42) are contained,
a means in the data processing system (21) for producing a relationship between the positional data of the measurement points (42) scanned by the co-ordinate measuring device (1,50) in the tomogram information and thus generally between positional data of the tomogram information and the attitude data of the surgical instrument (31) determined by the co-ordinate measuring device (1;50),
a means in the data processing system (21) for superimposing on the tomogram information the attitude data of the surgical instrument (31) ascertained during the surgical operation in the body part due to said relationship, and for producing superimposition images (43),
a means connected with the data processing system (21) for continuous reproduction of the superimposition images (43) on the display screen (22) which show continuously the positions of the surgical instrument in the tomograms (41),
a means connected with the data processing system (21) for substituting on the display screen (22) that tomogram (41) in which the surgical instrument (31) is located in a given moment with the tomogram (41) situated next to it when the surgical instrument (31) has moved from the area of this tomogram (41) to the area of the neighbouring tomogram (41).
a means in the data processing system (21) for comparing the momentary positional data of the measurement points (42) determined at time intervals by the co-ordinate measuring device (1;50) with the original positional data of the measurement points (42) and carrying out correction of the position of the tomograms (41) displayed on the output device when divergencies occur.

2. Device according to Claim 1, **characterised in that** the carrier (16) of the surgical instrument (31) is mounted on a three-dimensionally freely movable articulated arm (20; 72), and the coordinate measuring device (1;50) has synchros connected to the articulated arm (20; 72).

3. Device according to Claim 1, **characterised in that** the coordinate measuring device is constructed as a locator with contactless determination of the attitude data of the surgical instrument (31).

4. Device according to Claim 2, **characterised in that** the articulated arm (20; 72) contains six axes of rotation.

5. Device according to Claim 4, **characterised in that** the articulated arm (72) has a balancing mechanism (73).

6. Device according to Claim 5, **characterised in that** two members (61; 65, 68, 69) of the articulated arm (72) that can be pivoted about horizontal axes (62; 66) are connected via toothed belt drives (85, 86; 87, 88, 90) to their own counterweights (79, 81) in each case.

7. Device according to one or more of Claims 1 to 6 **characterised in that** the surgical instruments (31) and/or the probe can be connected via a coupling (32) to the coordinate measuring device (1; 50) and are provided with an identification code (34) and **in that** a code scanner (36) which is connected for the purpose of reproducing the code information to the data processing system (21) is arranged in the region of the coupling (32).

8. Device according to one or more of Claims 1 and 4 to 7, **characterised in that** the measuring instruments of the coordinate measuring device (1; 50) that are assigned to the individual articulations are arranged in a central housing and connected to the articulations by flexible drives and gear trains.

## Revendications

1. Dispositif destiné à la réalisation d'un procédé pour la représentation optique reproductible d'une intervention à effectuer à l'aide d'un instrument chirurgical (31), intervention avec laquelle
les informations tomographiques de la partie du corps dans laquelle l'intervention chirurgicale est à effectuer, sont mémorisées dans la mémoire de données d'un système de traitement des données (21),
les données relatives à la position du champ d'intervention sont déterminées à partir des informations tomographiques,
l'instrument chirurgical (31) est fixé sur un support tridimensionnel (16) à mouvement libre,
les données relatives à la position de l'instrument chirurgical (31) sont déterminées grâce à un dispositif de mesure des coordonnées (1;50) et transmises au système de traitement des données (21), qui établit une relation entre les données relatives à la position de l'instrument chirurgical (31) et les données relatives à la position du champ d'intervention, et
avec lequel l'instrument chirurgical (31) est orienté vers le champ d'intervention grâce à cette relation, dans quel cas
au moins trois points de mesure (42) accessibles à partir de l'extérieur sont déterminés ou repérés à titre de points de référence sur la partie du corps,
les tomographies (41) comprenant les points de mesure (42) sont effectuées de la partie du corps et stockées dans la mémoire de données,
la position dans l'espace des points de mesure (42) est détectée par le dispositif de mesure des coordonnées (1 ;50) et les données mesurées sont stockées dans la mémoire de données,
le système de traitement des données (21) établit une relation entres les données d'image des points de mesure (42) contenues dans les tomographies (41) et les données déterminées par le dispositif de mesure des coordonnées (1 ;50) à partir des points de mesure (42),
le dispositif de mesure des coordonnées (1 ;50) détecte en permanence la position dans l'espace de l'instrument chirurgical (31) tridimensionnel à mouvement libre, et les données de position sont transmises au système de traitement des données (21),
le système de traitement des données (21) superpose les données relatives à la position de l'instrument chirurgical (31) aux informations d'image des tomographies (41) et
le système de traitement des données (21) produit des images en superposition (43) sur un dispositif de sortie, en particulier un écran de visualisation (22), dans lesquelles les contenus des tomographies (41) et les positions respectives de l'instrument chirurgical (31) sont superposées,
le dispositif comprend :
un système de traitement des données (21),
une mémoire de données intégrée au système de traitement des données (21), dans laquelle les informations tomographiques d'une partie de corps sont stockées sous la forme d'une série de tomographies (41),
un écran de visualisation (22) relié au système de traitement des données (21) destiné à la restitution de tomographies (41) choisies à partir des informations tomographiques,
un support tridimensionnel (16) à mouvement libre, qui porte un instrument chirurgical (31),
un dispositif de mesure des coordonnées (1 ; 50) couplé au support (16) et au système de traitement des données (21), destiné à déterminer les données relatives à la position de l'instrument chirurgical (31), lesquelles peuvent être mises en relation avec des données de position contenues dans les informations topographiques par le système de traitement des données (21),
au moins trois points de mesure (42) déterminés sur la partie du corps et accessibles à partir de l'extérieur,
des informations tomographiques comprenant des points de mesure (42),
des moyens intégrés au système de traitement des données (21), destinés à établir une relation entre les données de position des points de mesure (42) explorés par le dispositif de mesure des coordonnées (1; 50) et les données de position des points de mesure (42) des informations tomographiques, et de ce fait, une relation généralisée entre les données de position des informations tomographiques et les données relatives à la position de l'instrument chirurgical (31) déterminées par le dispositif de mesure des coordonnées (1; 50),
des moyens intégrés au système de traitement des données (21), destinés à la superposition des informations tomographiques avec les données relatives à la position de l'instrument chirurgical (31) détectées grâce à la dite relation dans la partie du corps au cours d'une intervention chirurgicale, et à la production d'images en superposition (43), et
des moyens reliés au système de traitement des données (21), destinés à la restitution permanente des images en superposition (43) sur l'écran de visualisation (22), qui affiche en permanence les positions de l'instrument chirurgical (31) dans les tomographies (41),
des moyens reliés au système de traitement des données (21), destinés à échanger ensuite la tomographie (41), dans laquelle l'instrument chirurgical (31) se trouve actuellement, contre la tomographie (41) située à côté d'elle, quand l'instrument chirurgical (31) s'est déplacé du secteur de cette tomographie (41) dans le secteur de la tomographie (41) voisine, et
des moyens dans le système de traitement des données (21), destinés à comparer les données relatives à la position momentanée des points de mesure (42) et déterminées à des intervalles de temps par le dispositif de mesure des coordonnées (1;50), avec les données de position d'origine des points de mesure (42) et à procéder en cas de divergence à une correction de la position de la tomographie (41) représentée sur le dispositif de sortie.

2. Dispositif selon la revendication 1, **caractérisé en ce que** le support (16) de l'instrument chirurgical (31) est monté sur un bras articulé tridimensionnel à mouvement libre (20 ; 72), et **en ce que** le dispositif de mesure des coordonnées (1 ; 50) comporte des capteurs angulaires reliés au bras articulé (20 ; 72).

3. Dispositif selon la revendication 1, **caractérisé en ce que** le dispositif de mesure des coordonnées est conçu sous la forme d'un dispositif de repérage avec détermination sans contact des données relatives à la position de l'instrument chirurgical (31).

4. Dispositif selon la revendication 2, **caractérisé en ce que** le bras articulé (20 ; 72) comporte six axes de rotation.

5. Dispositif selon la revendication 4, **caractérisé en ce que** le bras articulé (72) comporte un mécanisme de compensation d'équilibre (73).

6. Dispositif selon la revendication 5, **caractérisé en ce que** deux éléments (61 ; 65, 68, 69) du bras articulé (72) pivotant autours d'axes horizontaux (62 ; 66), sont reliés par une transmission à courroie crantée (85, 86 ; 87, 88, 90) à leur propre masse de compensation respective (79, 81).

7. Dispositif selon l'une quelconque ou plusieurs des revendications 1 à 6 **caractérisé en ce que** les instruments chirurgicaux (31) et/ou le palpeur peuvent être reliés au dispositif de mesure des coordonnées (1 ; 50) au moyen d'un accouplement (32) et sont munis d'un code de repérage (34), et **en ce qu'**un lecteur de code (36), qui est relié au système de traitement des données (21) pour la transmission des informations de code, est disposé au niveau de l'accouplement (32).

8. Dispositif selon l'une quelconque ou plusieurs des revendications 1 et 4 à 7, **caractérisé en ce que** les instruments de mesure du dispositif de mesure des coordonnées (1; 50) qui sont associés aux différentes articulations, sont disposés dans un boîtier central et reliés aux articulations par des moyens d'entraînement et par des transmissions à roues dentées.
